# EUROPEAN PATENT APPLICATION

(11) **EP 4 443 384 A1**
(43) Date of publication of application: **09.10.2024**
(21) Application number: 24167982.8
(22) Date of filing: 02.04.2024
(51) Int. Cl.: G06T 7/00, G16H 30/40

(54) **METHOD AND SYSTEM FOR COMPARING PREVIOUS IMAGE AND CURRENT IMAGE**

(30) Priority: 06.04.2023 KR 20230045366; 16.11.2023 KR 20230159411
(71) Applicant: Lunit Inc., Seoul, 06241 (KR)
(72) Inventor: JUNG, Haein, 06241 Seoul (KR); HA, Yuri, 06241 Seoul (KR)
(74) Representative: Germain Maureau

(57) **Abstract**

Disclosed is a device for analyzing an image, the device including: a memory; and a processor for executing instructions stored in the memory. The processor is configured to analyze a target image of a patient by using an artificial intelligence model to obtain an analysis result including lesion information, and determine a previous image taken prior to the target image as a reference image, among images of the patient, and perform a comparative analysis on the reference image and the target image to obtain a comparative analysis result including a degree of lesion change.

## Description

### BACKGROUND

### (a) Field

The present disclosure relates to artificial intelligence-based medical image analysis technology.

### (b) Description of the Related Art

Previously, computer-aided detection (CAD) devices detected lesions in medical images on a rule basis, or detected lesions within specified candidate regions of the medical image. However, with the growing integration of artificial intelligence (AI) technology in healthcare, there has been a surge in AI-based medical image analysis technologies like the Lunit INSIGHT solution. These technologies leverage AI to analyzes medical images and presents the analysis results visually.

Users may identify abnormal lesions detected by the image analysis and perform tasks, such as reading and diagnosis. However, in cases where patients who require ongoing monitoring, users have to manually search for the patient's previous image, open the previous image, and visually compare the current image with the previous image to assess any changes in the lesion.

### SUMMARY

The present disclosure attempts to provide a method and a system for comparing a current image with a previous image.

The present disclosure attempts to provide a method and a system for providing a comparative analysis result between a target image and a previous image for a analysis result of a target image.

An exemplary embodiment of the present disclosure provides a device for analyzing an image, the device including: a memory; and a processor for executing instructions stored in the memory. The processor is configured to analyze a target image of a patient by using an artificial intelligence model to obtain an analysis result including lesion information, and determine a previous image taken prior to the target image as a reference image, among images of the patient, and perform a comparative analysis on the reference image and the target image to obtain a comparative analysis result including a degree of lesion change.

The processor may be configured to determine an image satisfying a condition among previous images of the patient as the reference image.

The processor may be configured to extract lesion information on at least one target lesion for comparison from an analysis result of the reference image and the analysis result of the target image, determine the degree of change for each target lesion over time based on the extracted lesion information, and generate the comparative analysis result including the degree of change for each target lesion.

The processor may be configured to determine the degree of change for each target lesion as one of No change, Disappeared, Appeared, Decreased, Increased, and No existence.

The processor may be configured to determine that a lesion change for a target lesion exists when the target lesion has been changed equal to or greater than a threshold for the target lesion.

The processor may be configured to determine the lesion change by using a threshold set for each comparison target lesion.

The processor may be configured to select a plurality of previous images taken prior to the target image from among the patient's images as a plurality of reference images, and perform a comparative analysis on temporally adjacent images in the plurality of reference images and the target image to obtain a plurality of comparative analysis results including the degree of lesion change between the adjacent images.

The processor may be configured to generate results of the analyses performed for the target image in a specified data format and store the results generated in the specified data format in an image storage device.

The processor may be configured to generate the comparative analysis result as a secondary capture of a DICOM standard.

The processor may be configured to generate an augmented secondary capture in which the comparative analysis result is appended to the analysis result of the target image.

The processor may be configured to generate an additional secondary capture that shows a comparison between an analysis result of the reference image and the analysis result of the target image.

Another exemplary embodiment of the present disclosure provides a method of operating an image analysis device, the method including: analyzing a target image of a patient by using an artificial intelligence model to obtain an analysis result including lesion information; and determining a previous image taken prior to the target image as a reference image, among images of the patient; and performing a comparative analysis on the reference image and the target image to obtain a comparative analysis result including a degree of lesion change.

The performing the comparative analysis may include: extracting lesion information on at least one target lesion for comparison from an analysis result of the reference image and the analysis result of the target image; determining the degree of change for each target lesion over time based on the extracted lesion information; and generating the comparative analysis result including the degree of change for each target lesion.

The determining the degree of change may include determining the degree of change for each target lesion as one of No change, Disappeared, Appeared, Decreased, Increased, and No existence.

The determining the degree of change may include, by using a threshold set for a target lesion, determining that a lesion change for the target lesion exists when the target lesion has been changed equal to or greater than a threshold for the target lesion.

The method may further include generating results of the analyses performed for the target image in a specified data format.

The specified data format may include a secondary capture of the DICOM standard. The generating the results of the analyses in the specified data format may include: generating an augmented secondary capture in which the comparative analysis result is appended to the analysis result of the target image or generating an additional secondary capture that shows a comparison between an analysis result of the reference image and the analysis result of the target image.

Still another exemplary embodiment of the present disclosure provides a computer program stored in a computer-readable recording medium, the computer program including instructions to cause a processor executing the computer program to display a worklist including a list of images for an image reading task in association with an image storage device and display analysis results including a comparative analysis result between a target image of a patient and a previous image taken prior to the target image among the images of the patient, upon responding to a selection of the target image of a patient is selected in the worklist.

The computer program may further include instructions to cause the processor to: display the comparative analysis result in the worklist. The comparative analysis result may include a degree of lesion change obtained by performing a comparative analysis on a current image to be read and a previous image taken prior to the current image.

The computer program may further include instructions to cause the processor to: display an augmented secondary capture in which the comparative analysis result is appended to an analysis result of the target image or display an additional secondary capture that shows a comparison between an analysis result of the previous image and the analysis result of the target image.

According to the exemplary embodiments, the comparative analysis result between the target image and the previous image is provided as the analysis result of the target image, so that the user can promptly understand the changes over time of the lesions detected in the target image without need to check the previous image separately.

According to the exemplary embodiments, the comparative analysis results with previous images may be integrated into a viewer, worklist, analysis report, or the like to improve the accuracy of the image interpretation and increase the efficiency of the image reading task for radiology.

According to the exemplary embodiments, through the augmented SC or additional SC, lesions that require attention or the degree of lesion change are visually distinguished and highlighted, thereby improving the accuracy of image interpretation and increasing the efficiency of image reading task.

According to the exemplary embodiments, the priority of the tasks in the worklist may be determined based on the comparative analysis result between the target image and the previous image, thereby increasing the efficiency of the image reading task.

### BRIEF DESCRIPTION OF THE DRAWINGS

FIG. 1 is a block diagram of a medical imaging system according to an exemplary embodiment.
FIG. 2 is a diagram illustrating an example of a graphical indicator representing the degree of lesion change according to the exemplary embodiment.
FIGS. 3 and 4 are diagrams illustrating examples of screens providing a result of a comparative analysis of a target image and a previous image according to the exemplary embodiment.
FIG. 5 is a diagram illustrating a sequence in which a result of a comparative analysis is output on a viewer according to the exemplary embodiment.
FIGS. 6 to 8 are diagrams illustrating examples of a screen providing an augmented SC according to the exemplary embodiment.
FIGS. 9 and 10 are diagrams illustrating examples of a screen providing an additional SC according to the exemplary embodiment.
FIGS. 11 and 12 are diagrams illustrating examples of a generated analysis report according to the exemplary embodiment.
FIG. 13 is a diagram illustrating an example of a generated worklist according to the exemplary embodiment.
FIG. 14 is a flow diagram of an image analysis method according to the exemplary embodiment.
FIG. 15 is a flow diagram of an image analysis method according to another exemplary embodiment.
FIG. 16 is a flow diagram of a method of providing an analysis result according to an exemplary embodiment.

### DETAILED DESCRIPTION OF THE EMBODIMENTS

Hereinafter, exemplary embodiments of the present invention will be described with reference to accompanying drawings so as to be easily understood by a person ordinary skilled in the art. The present disclosure can be variously implemented and is not limited to the following exemplary embodiments. In addition, in order to clearly explain the present description in the drawings, parts irrelevant to the description are omitted, and similar parts are denoted by similar reference numerals throughout the specification.

In addition, unless explicitly described to the contrary, the word "comprise", and variations such as "comprises" or "comprising", will be understood to imply the inclusion of stated elements but not the exclusion of any other elements. In addition, the terms "-er", "-or", and "module" described in the specification mean units for processing at least one function and operation, and may be implemented by hardware components or software components, and combinations thereof.

A device or a terminal of the present disclosure is a computing apparatus configured and connected so that at least one processor performs the operations of the present disclosure by executing instructions. A computer program may include instructions that cause a processor to execute the operations of the present disclosure and may be stored on a non-transitory computer readable storage medium. The computer program may be downloaded through a network or sold as a product.

Medical images of the present disclosure may be images of various parts of the body taken (photographed or captured) with various modalities, for example, the modalities may be x-rays, magnetic resonance imaging (MRI), ultrasound, computed tomography (CT), digital mammography (MMG), and digital breast tomosynthesis (DBT).

A user in the present disclosure is a healthcare professional, and may include, but is not limited to, a physician, nurse, clinical pathologist, sonographer, or medical imaging professional (a radiologist).

An artificial intelligence model (AI model) of the present disclosure may be a machine learning model for learning at least one task, which may be implemented as a computer program executable by a processor. The task that an AI model learns may refer to the task desired to be solved with machine learning, or the task desired to be performed with machine learning. The AI model may be implemented as a computer program that runs on a computing device, and may be downloaded through a network, or sold as a product. Alternatively, the AI model may work with a variety of devices through a network.

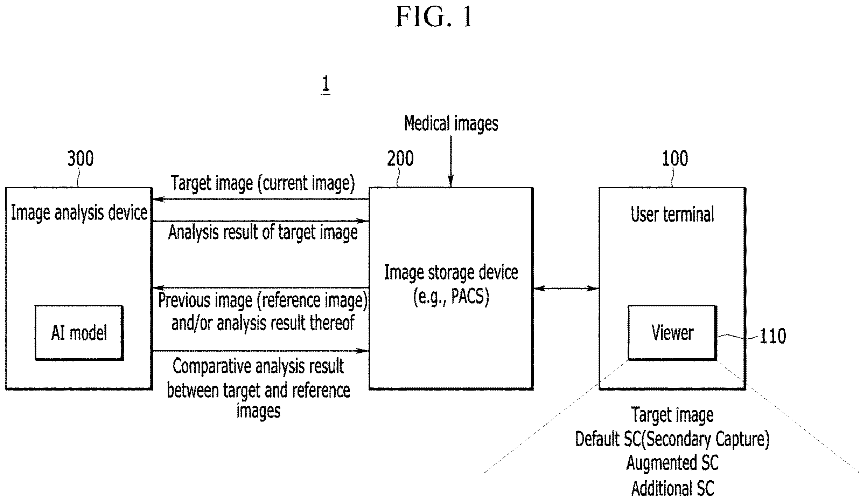

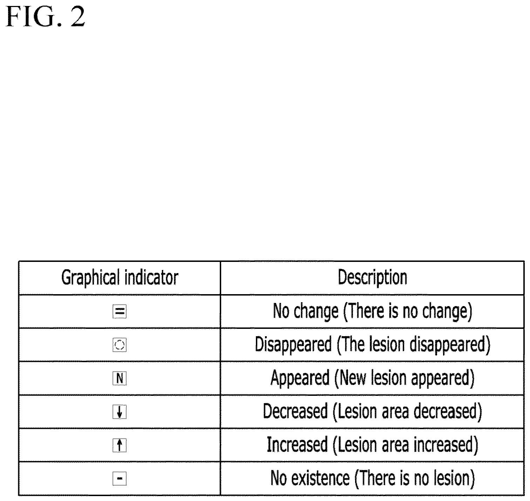

FIG. 1 is a block diagram of a medical imaging system according to an exemplary embodiment, and FIG. 2 is a diagram illustrating an example of a graphical indicator representing the degree of lesion change according to the exemplary embodiment.

Referring to FIG. 1, a medical imaging system 1 may include at least one user terminal 100, an image storage device 200, and an image analysis device 300.

The user terminal 100 includes hardware and software that installs programs executed by a processor and provides a computing environment and network environment for performing the operations of the present disclosure. The user terminal 100 may be implemented in various types, such as, for example, a computing device in a workstation, or a mobile device. The user terminal 100 may include a viewer (simply referred to as "viewer") 110 that is associated with the image storage device 200 to display medical image-related data stored in the image storage device 200. The viewer 110 may be installed and executed on, for example, a computing device within a workstation, may be implemented to access the image storage device 200, and may display medical image-related data stored on the image storage device 200. The viewer 110 is a computer program stored on a computer-readable medium and includes instructions executed by a processor. The processor of the user terminal 100 may execute the instructions to perform the operations described herein.

The viewer 110 may display an image analysis result stored in the image storage device 200. The viewer 110 may provide a worklist arranged in a tabular format and show a list of images assigned for the user's reading task along with essential details. The viewer 110 may include a picture archiving and communication system (PACS) viewer. Here, the viewer 110 is a program designed to display image analysis result stored in the image storage device 200 and may support image reading task associated with the worklist, but is not necessarily limited to a viewer for reading work.

The image storage device 200 may store and manage medical images. The image storage device 200 may store and manage analysis results for the medical images. The image storage device 200 may include a PACS database. The image storage device 200 may store data according to a specified data format. For example, the image storage device 200 may store medical images taken by medical imaging devices in accordance with the digital imaging and communications in medicine (DICOM) standard, and the results of analyzing the medical images, and may communicate with the user terminal 100 to provide data for image reading. The image storage device 200 and the viewer 110 may be configured as a PACS system, and the image storage device 200 may be a PACS server/DB and the viewer 110 may be a PACS viewer.

The DICOM standard utilized for medical image storage is described herein as an example, but the medical image standard needs not be limited to DICOM.

The image storage device 200 may obtain an analysis result of the medical image from the image analysis device 300. The analysis result of the medical image may include lesion information and various medical predictions. The analysis result of the medical image may be provided to assist a user for image reading task, and may be provided as an auxiliary image displaying lesion information on the image. For example, the auxiliary image may be a secondary capture (SC) image of DICOM (simply referred to as SC). The SC image is a separate image from the original medical image, generated by displaying lesion information on the original medical image, and may be displayed in the PACS viewer. The analysis result of the medical image may also be provided as a textual report. For example, the report may be provided according to a DICOM Basic Text Structured Report (SR). However, the form of providing the analysis results is not limited thereto and may include results in various DICOM formats.

The image storage device 200 may obtain, from the image analysis device 300, a comparative analysis result between a target image and a previous image. The comparative analysis result between the target image and the previous image may include the degree of lesion change obtained by comparing the analysis result of the target image with the analysis result of the previous image. The comparative analysis result may also be provided to assist the user for image reading, which may be provided in various ways. For example, the comparative analysis result may be provided together with the default SC of the target image, which may be referred to as an augmented SC. Alternatively, the comparative analysis result may be provided as a separate SC from the default SC of the target image, which may be referred to as an additional SC. The augmented SC and the additional SC are described in more detail in the following sections

The medical images stored in the image storage device 200 may include images obtained by medical image devices of various modalities. The medical image may be x-ray images, magnetic resonance imaging (MRI) images, ultrasound images, computed tomography (CT) images, digital mammography (MMG) images, digital breast tomosynthesis (DBT) images, and the like. The present disclosure describes a chest x-ray image as an example of a medical image, but the medical image needs not be limited thereto, and the present disclosure may be adapted to any type of medical image.

The image analysis device 300 may analyze the medical image by using an artificial intelligence (AI) model, and store the analysis result in the image storage device 200. The image analysis device 300 may be equipped with an AI model specialized for each type of medical image, and may select an AI model according to the type of input image to perform an analysis, such as lesion detection, suitable for the input image. The AI model is generated to make medical inferences from the input medical image. The model structure, training data, training method, and medical inference target may be designed in various ways.

Further, the image analysis device 300 may compare and analyze the image requested for analysis (the target image) with the previous image, and store the comparative analysis result between the target image and the previous image in the image storage device 200. For the comparative analysis, the image analysis device 300 may extract at least one previous image related to the target image, and perform a comparative analysis of the target image and the previous image. Comparative analysis may be performed to images taken at different time points, which may include at least one previous image to be compared with the current image. Here, the medical image for analysis may be referred to as a target image or the current image, and the previous image of the patient taken prior to the current image may be referred to as a reference image. In the description, single previous image is mainly used for the reference image, but a plurality of previous images may be used for the reference image for comparison.

When the image analysis device 300 receives an analysis request for a target image of a patient, the image analysis device 300 may analyze the target image to obtain an analysis result including lesion information. Further, when the image analysis device 300 receives an analysis request or a comparative analysis request for the target image, the image analysis device 300 may identify a previous image of the patient that was taken prior to the target image, and compare and analyze the previous image and the target image to obtain a comparative analysis result, such as the degree of lesion change. For example, the image analysis device 300 may search for previous images taken prior to the target image in the image storage device 200 based on a patient ID of the target image, and perform a comparative analysis of the target image and the previous images. Alternatively, the analysis request may include information about the previous images to be used in the comparative analysis so that the image analysis device 300 does not need to search for the previous images. In the description, it has been described as the image analysis device 300 determines the previous images. Alternatively, the previous images to be compared may be specified by user input, or the image storage device 200 may inform the image analysis device 300 of previous images selected based on criteria from among homogeneous images of the same patient.

The previous image that serves as the reference for comparison may be determined in a variety of ways. For example, the image analysis device 300 may determine the most recent image of the patient's previous images stored in the image storage device 200 as the reference image. The image analysis device 300 may search for previous images of the patient taken within a specified time period (e.g., the last n days, the last n months, or the last n years) relative to the photographing date of the target image. Subsequently, the image analysis device 300 may determine the most recent image among the previous images searched or an image satisfying a specific condition as the reference image for comparison. The condition may include selecting an image taken before a certain period (e.g., 3 months) from the photographing date of the target image to detect significant changes, or selecting images that have undergone AI analysis or reading. Various other conditions may be used to determine the previous images to be used for comparative analysis. On the other hand, the images taken by the imaging device are automatically archived in the image storage device 200. In instances where images (e.g., x-ray images) are inaccurately taken and immediately reshoot, duplicate or inaccurate images may accumulate in the image storage device 200. Therefore, to mitigate the possibility of mistakenly identifying images taken at similar times to the target image as the previous image, the image analysis device 300 may disregard consecutive images taken before the target image when selecting the reference image. The consecutively taken images may be, for example, images taken with a minute-level time difference from the target image, and the exclusion criteria may be set in various ways.

The image analysis device 300 may perform the comparative analysis in a variety of ways. For example, the image analysis device 300 may analyze both the previous image and the current image of the patient, detect lesions in each of the two images, and detect the degree of change in the lesions. Alternatively, when the image analysis device 300 has already analyzed the previous image, the image analysis device 300 may extract lesion information from the analysis result of the previous image and compare the lesion information with the lesion information detected in the current image to detect the degree of lesion change. To simplify the explanation, it may be described that during the comparative analysis of the target image and the previous image, the previous image and the current image are compared and analyzed, which may include utilizing analysis results of previous images that have already been performed.

The timing of the comparative analysis by the image analysis device 300 between the target image and the previous image may be varied. For example, upon receiving an analysis request for a target images, the image analysis device 300 may analyze the target image, perform a comparative analysis between the target image and the previous image, and provide bath the comparative analysis result and the analysis result of the target image. Alternatively, upon receiving a request for a comparative analysis with a previous image, the image analysis device 300 may perform a comparative analysis between the target image and the previous image and provide the comparative analysis result between. The analysis request for the target image or the comparative analysis request with the previous image may be originated from the image storage device 200, be initiated from the user terminal 100, or even be automatically generated by the image analysis device 300 upon detecting the capture of the target image.

The following description will be given by using a chest x-ray image as an example.

The image analysis device 300 may analyze a chest X-ray image to detect lesions in the chest X-ray image. The lesions detectable in the chest x-ray image may include nodules, pneumoperitoneum, pleural effusion, consolidation, cardiomegaly, atelectasis, fibrosis, calcification, fibrosis, mediastinal widening, tuberculosis, acute bone fracture, and the like, as shown in Table 1. Further, the image analysis device 300 may analyze the chest x-ray image to obtain essential medical indicators, including an abnormality score. For example, the image analysis device 300 may infer an abnormality score, a tuberculosis (TB) analysis score, a cardiomegaly probability, and the like from the input image. Of course, the detectable lesions and medical indicators may vary depending on the images analyzed by the image analysis device 300.

**(Table 1)**

| Abbreviation | Lesion name |
|---|---|
| Ndl | Nodule |
| Ptx | Pneumothorax |
| PEf | Pleural effusion |
| Csn | Consolidation |
| Cm | Cardiomegaly |
| Atl | Atelectasis |
| Ppm | Pneumoperitoneum |
| Calc | Calcification |
| Fib | Fibrosis |
| MW | Mediastinal Widening |
| TB | Tuberculosis |
| Fx | Acute Bone fracture |

The lesions selected for comparison with the previous images, termed as the target lesions for comparison, may be set with all lesions detectable in the images. Alternatively, the target lesions for comparison may be set with some lesions among the lesions detectable in the image. The target lesions for comparison may be changed, and may be set differently depending medical institutions or medical professionals. For example, comparable lesions in the chest x-ray image may be predefined as shown in Table 2, which may be customizable. When the target lesions for comparison may be set differently by different medical practitioners, the image analysis device 300 may analyze the degrees of change for all selectable lesions by the user and display only the degrees of change for the selected lesions based on the user's configuration on the viewer 110.

**(Table 2)**

| Abbreviation | Lesion name |
|---|---|
| Ndl | Nodule |
| Ptx | Pneumothorax |
| PEf | Pleural effusion |
| Csn | Consolidation |

The image analysis device 300 obtains a comparative analysis result by assessing the changes in the lesions detected between the previous image and the current image. The image analysis device 300 may determine that there is a lesion change (increase or decrease) when the change equals or exceeds a threshold, the lesion in the target image being increased or decreased equal to or greater than the threshold. Conversely, the image analysis device 300 may determine that there is no lesion change when the change falls below the threshold. The threshold may be set differently for different types of lesions.

The method of comparing lesion change may vary. The lesion change may be determined by a change in the lesion area. The lesion change may be determined based on the ratio of the lesion area in the current image to the lesion area in the previous image (e.g., 1.2 times or 0.8 time). The lesion change may be determined by comparing the ratio of the area of the non-normal area (e.g., left lung nodule) to the reference area (e.g., left lung) calculated from each of the previous image and the current image. The lesion change may be determined by comparing the ratio of the abnormality score of the non-normal area (e.g., left lung nodule) to the abnormality score for the reference area (e.g., left lung) calculated from each of the previous image and the current image. The present disclosure does not need to be limited thereto.

The image analysis device 300 may determine the degree of lesion change, as shown in Table 3.

**(Table 3)**

| Lesion change | Explanation |
|---|---|
| No change | No change in lesion area. |
| Disappeared | Lesion was detected in the previous images, but not in the current image. |
| Appeared | Lesion was not detected in the previous image, but is detected in the current image |
| Decreased | Lesion area is smaller in the current image than in the previous image |
| Increased | Lesion area is larger in the current image than in the previous image. |
| No existence | Lesion is not detected in both the previous image and the current image. |

When a lesion is present in both previous and current images, but remains unchanged in size over time, the comparative analysis result for the corresponding lesion is determined as `No change'. When a lesion is detected in the previous image but not detected in the current image, the comparative analysis result for the corresponding lesion is determined as 'Disappeared'. When a lesion is not detected in the previous image but is detected in the current image, the comparative analysis result for the corresponding lesion is determined as 'Appeared'. When the lesion area is smaller in the current image than in the previous image, the comparative analysis result for the lesion is determined as 'Decreased'. When the lesion area is increased in the current image compared to the previous image, the comparative analysis result for the lesion is determined as 'Increased'. In this case, the image analysis device 300 may calculate a ratio of the lesion area (e.g., the nodule area of the left lung) relative to the reference area (e.g., the left lung), and determine an increase or decrease in the lesion area by comparing the area ratios. Alternatively, the image analysis device 300 may calculate a ratio of an abnormality score for the lesion area (e.g., the nodular area of the left lung) relative to an abnormality score for the reference area (e.g., the left lung), and determine an increase or decrease in the lesion area by compare the score ratios. When the lesion is not detected in both the previous image and the current image, the comparative analysis result for the lesion is determined as `No existence'.

In another exemplary embodiment, the image analysis device 300 may perform a comparative analysis between a plurality of previous images and a current image. The number N of medical images to be used in the comparative analysis and an image selecting method may be predefined by user's configurations or may be adaptively determined during the progress course of the comparative analysis.

When N images are selected from the patient's images, the image analysis device 300 may generate a comparative analysis result by comparing and analyzing each pair of images to determine the degree of lesion change. By comparing two temporally adjacent images, the image analysis device 300 may obtain N-1 results of the comparative analysis. The N-1 results of the comparative analysis may be presented as SCs including the degree of lesion change. The image analysis device 300 may then synthesize the N-1 results of the comparative analysis to generate an overall comparative analysis result. The overall comparative analysis result may be expressed, for example, as information indicating a trend of the lesion change, such as a change rate, a change graph overtime (photographing date), a table, or the like. The overall comparative analysis result may be provided as a distinct report or interface.

The image analysis device 300 may adaptively select previous images. According to one exemplary embodiment, when the lesion has disappeared, appeared, or has no change as a result of the comparison between the current image and the immediate previous image (previous image#1), the image analysis device 300 may determine it unnecessary to check the previous image of the immediate previous image. However, when the lesion has increased or decreased, the image analysis device 300 may search for the initial previous image (e.g., previous image# n) where the corresponding lesion initially appeared using the results of the analysis or reading report of the previous images. the image analysis device 300 may then compare and analyze the images from the initial previous image to the current image to analyze the lesion change between adj acent images, and analyze a change trend of the corresponding lesion (change rate, change graph over time, table, and the like). Moreover, the image analysis device 300 may select at least a subset of the images from the initial previous image to the current image based on user selection or selection criteria (e.g., a photographing interval of three months or more) and compare and analyze the selected images.

According to another exemplary embodiment, the image analysis device 300 may analyze a certain number of previous images sequentially when the lesion has increased or decreased as a comparison result with the immediate previous image (previous image #1). For example, when the image analysis device 300 is set to analyze up to three previous images, the image analysis device 300 may search for a previous image (previous image #2) of the immediate previous image #1, and when the analysis result between previous image #2 and previous image #1 also show an increase/decrease in the lesion, the image analysis device 300 may search for a previous image (previous image #3) of previous image #2, and compare and analyze the previous image #3 and the previous image #2. As a result, the image analysis device 300 may analyze the lesion change between adjacent images by comparing and analyzing the images from the previous image #3 to the current image, and may also analyze the change trend of the corresponding lesion. However, if the analysis result between the previous image #2 and the previous image #1, indicate either a lesion appeared or no change, the image analysis device 300 may discontinue the search for the subsequent previous image #3, and may use the previous image #2 and the previous image #1 as the reference image.

The analysis result of the target image analyzed by the image analysis device 300 may be stored in the image storage device 200 and displayed on the user terminal 100. The analysis result may include a result of the AI analysis for the target image and a comparative analysis result between the target image and the previous image. When the image is a DICOM image, the metadata of the image may be stored in a Public tag and a Private tag. The Public tag contains information on the medical image following the file structure of the DICOM standard. Conversely, the private tag may allow medical device manufacturers to supplement the DICOM image with additional information not covered in the Public tag. So, the analysis result may be recorded in the Private tag. The private tag may record the result of the AI analysis including lesion information, and the comparative analysis result including the degree of lesion change, and may further include whether the current-prior image comparison function is activated.

The viewer 110 executed on the user terminal 100 may display the analysis result of the target image and the comparative analysis result between the target image and the previous image. The analysis result of the target image may be provided as a default SC. The comparative analysis result may be integrated into the default SC, and the default SC that further includes the comparative analysis result may be called an augmented SC. The comparative analysis result between may be provided as an separate SC from the default SC, and the separate SC provided in addition to the default SC may be referred to as an Additional SC.

The viewer 110 may display various information contained in the comparative analysis result. To facilitate this, the comparative analysis result may be stored in a DICOM format, a data format compatible with the viewer 110.

The viewer 110 may display the photographing date and time of the previous image and the current image.

The viewer 110 may display the degree of change for each of the comparison target lesions based on the comparative analysis result. The viewer 110 may display a lesion location that has a change among the target lesions for comparison. The viewer 110 may display lesions with a change (e.g., `Appeared', or 'Increased') warranting the attention in reading task to be distinguished from other lesions. For example, the viewer 110 may visually display the corresponding lesion or the degree of lesion change differently or with a highlight mark. in the case of chest x-ray images, the viewer 110 may display the degree of lesion change separately for the left lung and the right lung. The viewer 110 may provide a threshold used to determine the degree of lesion change.

Referring to FIG. 2, the viewer 110 may display the degree of lesion change included in the comparative analysis result as graphical indicators, such as icons. The graphical indicator assigned to each degree of change allows the user to intuitively and promptly recognize the lesion change.

The comparative analysis function may be utilized as follows.

For example, it is assumed that as a analysis result of a patient's chest x-ray, an abnormal lesion is detected and a patient undergoes a lung procedure. Subsequently, the doctor may request chest X-ray imaging through the hospital system to monitor the patient's progress. When the radiologist takes the chest X-ray image of the patient, the image is transmitted from the X-ray imaging device to the image storage device 200. The image analysis device 300 then compares and analyzes the chest X-ray images taken before and after the procedure to obtain a comparative analysis result including the degree of lesion change, and stores the comparative analysis result in the image storage device 200. Using the current-prior image comparison function of the image analysis device 300, the doctor may monitor the lesion changes before and after the procedure.

For example, it is assumed that as a analysis result of a patient's chest x-ray image, an abnormal lesion is detected and a doctor diagnoses that the lesion needs to be monitored. After a certain period, the patient undergoes another X-ray imaging, and the image is transmitted from the X-ray device to the image storage device 200. The image analysis device 300 then compares and analyzes the previously taken chest X-ray image and the currently taken chest X-ray image to obtain a comparative analysis result including the degree of lesion change, and stores the comparative analysis result in the image storage device 200. Using the current-prior image comparison function of the image analysis device 300 associated with the image storage device 200, the doctor may monitor lesion changes over time.

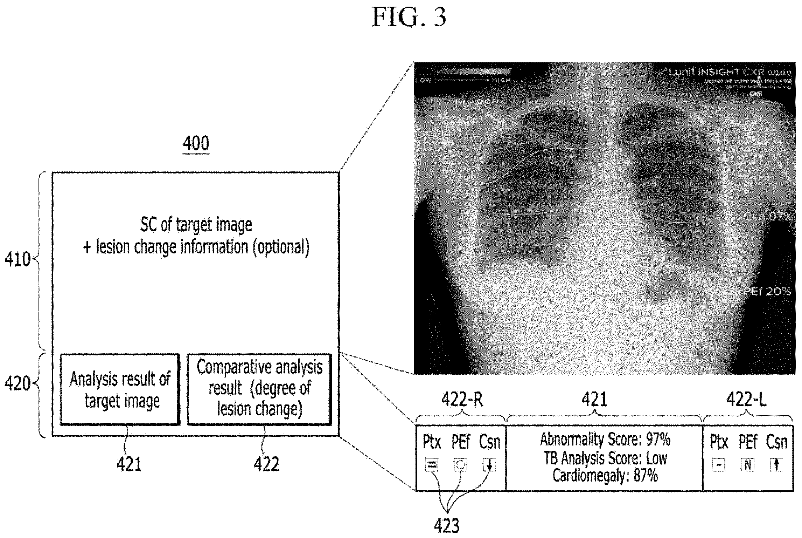

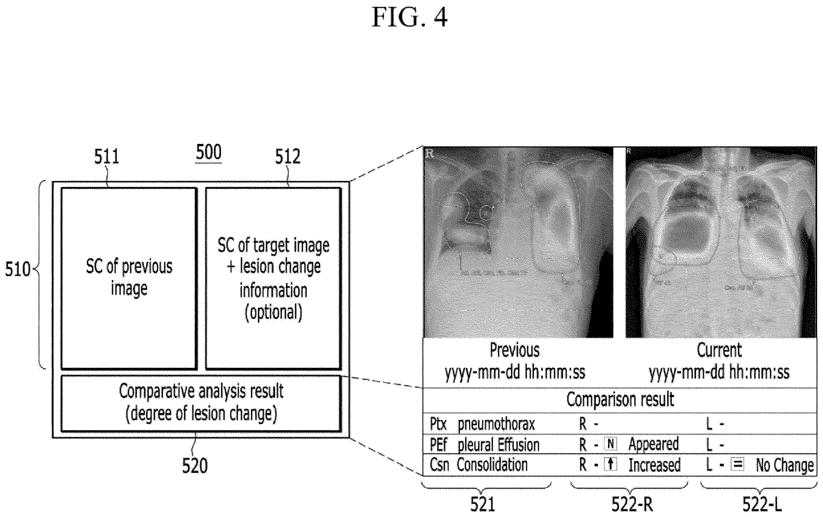

FIGS. 3 and 4 are diagrams illustrating examples of screens providing a result of a comparative analysis of a target image and a previous image according to the exemplary embodiment.

Referring to FIG. 3, the viewer 110 executed on the user terminal 100 may provide a screen related to a reading task of the target image in association with the image storage device 200, and may provide an output of a current-prior image comparison function on the screen. The current-prior comparison function may be manually selected by a user, or may be provided as a default setting. Upon activation of the current-prior comparison function in the image analysis device 300, the viewer 110 may display the comparative analysis result between the target image and the previous image on the screen, and may provide an augmented SC in which the comparative analysis result is included in the analysis result of the target image.

An augmented SC screen 400 displayed on the viewer 110 may include an image region 410 and an analysis information region 420. The image region 410 may display the default SC that includes the analysis result of the target image. The default SC may display the analysis result of the target image such as a lesion score like Ptx 88% or Csn 94%, a contour of the lesion area, and a heatmap. The lesion change information may be additionally displayed on the default SC. The lesion score may be a score/probability representing a confidence level that a lesion exists.

The analysis information region 420 may include a region 421 where the analysis result of the target image is displayed, and an region 422 where the comparative analysis result between the target image and the previous image (e.g., the degree of lesion change) is displayed. The region 421 may display medical indicators obtained by analyzing the target image with the AI model, for example, abnormality score, TB analysis score, or Cardiomegaly. The region 422 may display the degree of changes in the target lesions (e.g., Ptx, PEf, or Csn). When the right and left sides of an object are separated, such as in a chest x-ray image, the region 422 may separately display the degree of lesion change in right lung 422-R and the degree of lesion change in left lung 422-L. In this case, the degrees of change in the target lesions (e.g., Ptx, PEf, or Csn) may be displayed with graphical indicators 423, such as icons. Text describing the degree of change (e.g., `No change', and 'Disappeared') may be displayed or omitted depending on the size of the analysis information region 420. Even without textual descriptions, such as `No change', the user may intuitively and promptly recognize the degree of lesion change through graphical indicators, such as icons.

The augmented SC is a form of providing the comparative analysis result with the analysis result of the target image. It is necessary for the user to check the photographing date of previous images used in the comparative analysis to ascertain the change rate and the like. To this end, the augmented SC screen 400 may provide the photographing timing of the previous image used in the comparative analysis. Additionally, the augmented SC screen 400 may provide information on the time gap between the target image and the previous image (e.g., a comparison to the patient's image taken 8-week prior).

Referring to FIG. 4, upon activation of the current-prior comparison function in the image analysis device 300, the viewer 110 may display the comparative analysis result between the target image and the previous image on the screen, which may be provided through the additional SC.

The additional SC screen 500 displayed by the viewer 110 may include an image region 510 and an analysis information region 520. To allow a user to simultaneously check the previous image and the current target image, an SC 511 that includes the analysis result of the previous image and an SC 512 that includes the analysis result of the target image may be displayed together in the image region 510. The placement of the previous image and the target image may vary. The SCs 511 and 512 of the previous image and the target image may include the results of the analysis of the corresponding images (lesion information), respectively. The lesion change information may be further displayed in the SC of the target image.

The viewer 110 may provide information about the previous image and the target image such as photographing timing, and the like. The viewer 110 may provide information on the time gap between the target image and the previous image (e.g., comparison to the patient's image taken 8-week prior).

The analysis information region 520 displays the comparative analysis result (the degree of lesion change) with the previous image. The UI for displaying the degrees of changes in the target lesions (e.g., Ptx, PEf, or Csn) in the analysis information region 520 may be designed in a variety of ways, and for example, the lesion name 521 and the degree of change thereof may be displayed in the form of a table. When the right and left sides of the object are separated, such as in a chest x-ray image, the degree of lesion change in right lung 522-R and the degree of lesion change in left lung 522-L may be separated. In this case, the degrees of change for the target lesions (e.g., Ptx, PEf, or Csn) may be displayed as graphical indicators, such as an icon. The user may intuitively and promptly recognize the degree of lesion change through graphical indicators, such as icons. On the other hand, when there is space for writing text describing the degree of change (`No change', 'Disappeared', and the like), the text description may be displayed along with the graphical indicator.

As such, the additional SC provides the analysis results of the current image and the previous image on a single screen. The additional SC enables the user to easily compare the lesions detected in the two images directly on the additional SC screen 500 without need to refer the previous image of the patient on a separate screen.

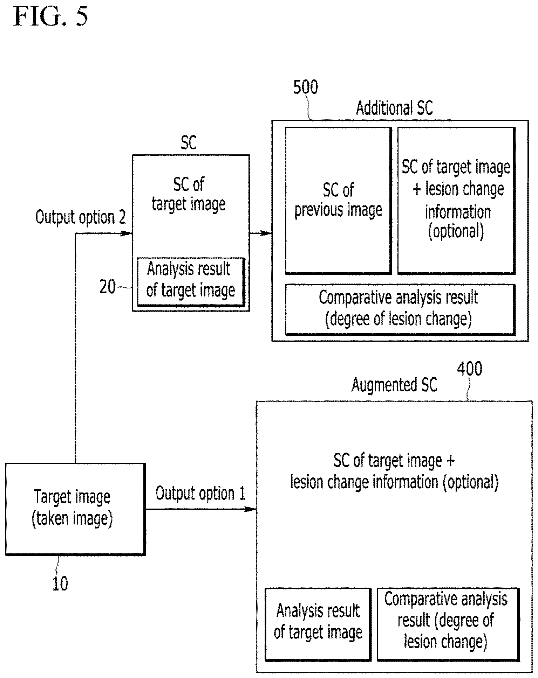

FIG. 5 is a diagram illustrating a sequence in which a result of a comparative analysis is output on a viewer according to the exemplary embodiment.

Referring to FIG. 5, the viewer 110 may work in association with the image storage device 200 to sequentially provide images for reading the target image. While a conventional viewer provides the taken image 10 and an SC 20 containing the analysis result of the target image, the viewer 110 of the present disclosure may further provide the comparative analysis result between the target image and the previous image.

When it is set to provide the comparative analysis result through the augmented SC, the viewer 110 may sequentially display the taken image 10 of the target image and an augmented SC 400. The augmented SC 400 may include the analysis result of the target image and the comparative analysis result.

When it is set to provide the comparative analysis result through the additional SC, the viewer 110 may sequentially display the taken image 10 of the target image, a default SC 20 with the analysis result of the target image, and an additional SC 500 with the comparative analysis result.

The output options for presenting the comparative analysis result via the viewer 110 may either be predetermined, or be selected by the user.

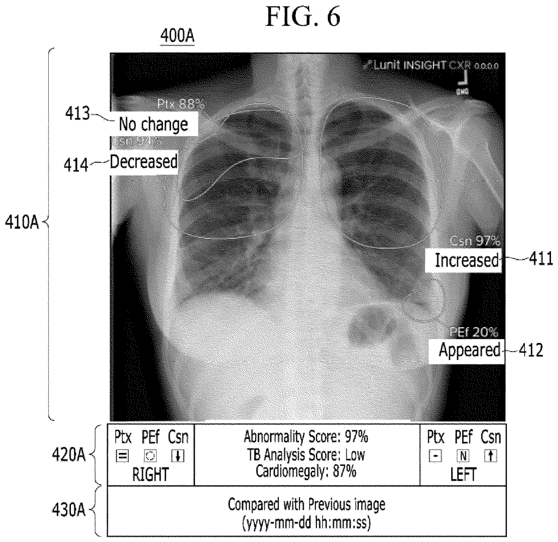

FIGS. 6 to 8 are diagrams illustrating examples of a screen providing an augmented SC according to the exemplary embodiment.

Referring to FIG. 6, the augmented SC screen 400a displayed on the viewer 110 may include an image region 410A and an analysis information region 420A, and may further include a region 430A displaying previous image information used for the comparative analysis. The arrangement or area size of the augmented SC screen 400A may be designed in a variety of ways.

In the image region 410A, an SC including the analysis result of the target image may be displayed. The SC displays the analysis result of the target image (e.g., lesion scores such as Ptx 88% and Csn 94%, contours of the lesion area, or heat maps), and may further display lesion change information 411, 412, 413, and 414 of the target lesions for comparison among the lesions detected in the target image (e.g., Csn and PEf in the left lung, Ptx and Csn in the right lung). The lesion change information 411, 412, 413, and 414 indicates the degree of lesion change ('Increased', `Appeared', `No change', 'Decreased') according to the comparative analysis result between the target image and the previous image, which may be displayed as text or displayed as a change degree icon.

The analysis information region 420A may display the analysis result of the target image, such as Abnormality Score, TB analysis score, and the like, and the comparative analysis result with the previous image, such as the degree of lesion change. In this case, the degrees of change for the target lesions (e.g., Ptx, PEf, or Csn) may be displayed with graphical indicators, such as icon, and the text describing the degree of change may be omitted or displayed.

The region 430A may display previous image information used in the comparative analysis. The previous image information may include the photographing timing of the previous images used in the comparative analysis or the time gap between the target image and the previous image.

The augmented SC screen 400A may identify a lesion changed (e.g., `Appeared' or 'Increased') that require further user attention and highlight the lesion for visual distinction. For example, because a lesion PEf has appeared in the left lung, the newly appeared lesion PEf, the lesion change information 412 of the newly appeared lesion PEf, or the change degree icon of the newly appeared lesion PEf may be displayed differently in color, size, shape, or the like, to be highlighted or a highlight mark may be added thereto.

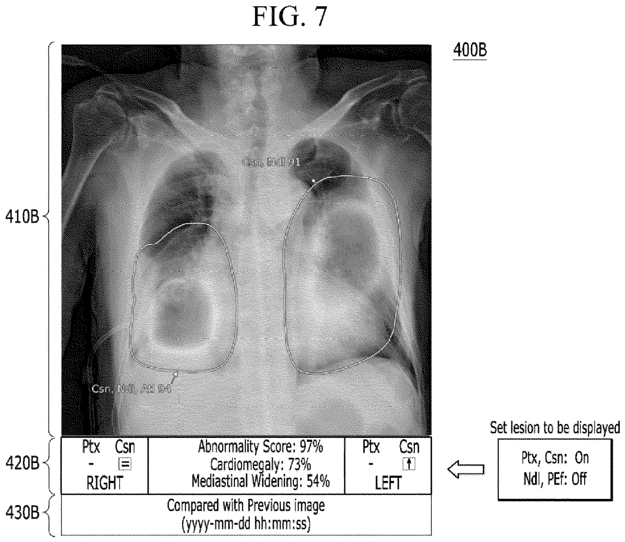

Referring to FIG. 7, the augmented SC screen 400B displayed on the viewer 110 may include an image region 410B, and an analysis information region 420B, and may further include a region 430B displaying previous image information used in the comparative analysis. The arrangement or area size of the augmented SC screen 400B may be designed to vary.

The image region 410B may display an SC including an analysis result of a target image. The SC may display an analysis result of a target image and may further display lesion change information of target lesions for comparison among the lesions detected in the target image, as illustrated in FIG. 6.

The analysis information region 420B may display the analysis result of the target image, such as Abnormality Score, TB analysis score, and the like, and the comparative analysis result with the previous image, such as the degree of lesion change. In this case, the degrees of change for the target lesions (e.g., Ptx, PEf, or Csn) may be displayed with graphical indicators, such as icon, and the text describing the degree of change may be omitted or displayed.

Further, depending on user's configuration, the c target lesions for omparison displayed on the augmented SC screen 400B may vary. For example, when the user sets Ptx and Csn for display while setting not to display Ndl and PEf, the analysis information region 420B may only display the degrees of change in Ptx and Csn, the lesions designated for display. In addition, the lesion change information displayed in the SC may only be provided for the target lesions selected for display.

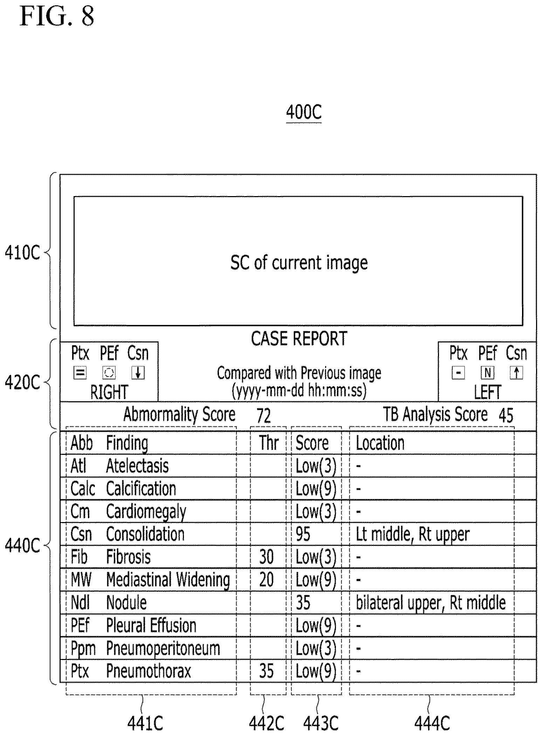

Referring to FIG. 8, the augmented SC screen 400C displayed on the viewer 110 may include an image region 410C and an analysis information region 420C, and may further include a detailed analysis table 440C. The layout or area size of the augmented SC screen 400C may be designed to vary.

The image region 410C may display an SC that includes the analysis result of the target image (current image). The secondary image SC may display the analysis result of the target image and may further display lesion change information of the target lesions for comparison among the lesions detected in the target image, as illustrated in FIG. 6.

The analysis information region 420C may display the analysis result of the target image, such as Abnormality Score, TB analysis score, and the like, and the comparative analysis result with the previous image, such as the degree of lesion change. In this case, the degrees of change for the target lesions (e.g., Ptx, PEf, or Csn) may be displayed with graphical indicators, such as icon, and the text describing the degree of change may be omitted or displayed.

The detailed analysis table 440C may include a lesion name 441C, a lesion-specific threshold 442C, a lesion score 443C, and a lesion location 444C, but may be varied.

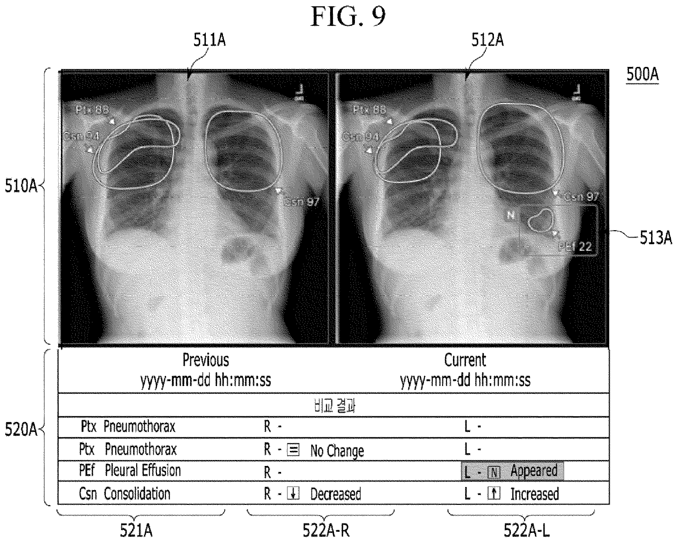

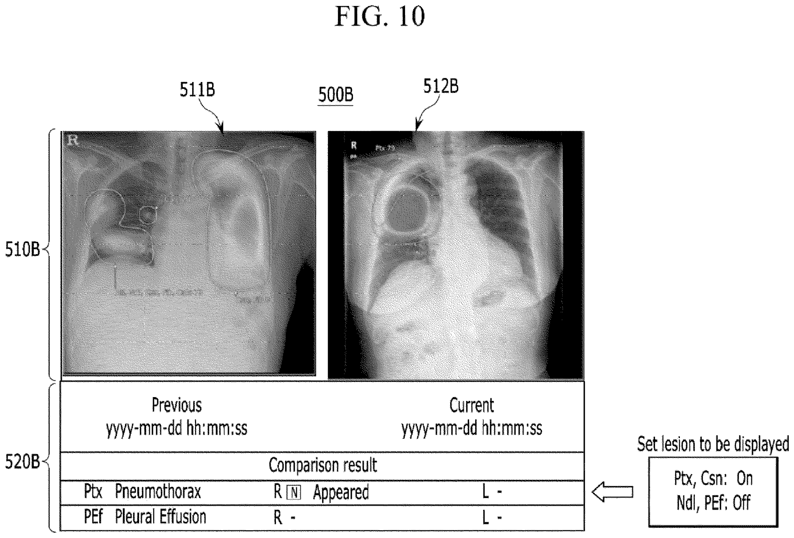

FIGS. 9 and 10 are diagrams illustrating examples of a screen providing an additional SC according to the exemplary embodiment.

Referring to FIG. 9, an additional SC screen 500A displayed on the viewer 110 may include an image region 510A and an analytical information region 520A, and may display photographing time points of a previous image and a target image. The arrangement or area size of the additional SC screen 500A may be designed to vary.

The image region 510A may display an SC 511A including an analysis result of the previous image and an SC 512A including an analysis result of the target image. The SCs 511A and 512A of the previous image and the target image may display the results of the analysis (lesion information) of the respective images, and the SC 512A of the target image may further display lesion change information of the target lesions for comparison among the detected lesions. The lesion change information may be displayed in various forms.

The analysis information region 520A displays the comparative analysis result with the previous image, such as the degree of lesion change. The UI for displaying the degrees of changes in the comparison target lesions (e.g., Ptx, PEf, or Csn) in the analysis information region 520A may be designed in a variety of ways, and for example, the lesion name 521A and the degree of change thereof may be displayed in the form of a table. When the left and right sides of the object are separated, such as in a chest x-ray image, the degree of lesion change in right lung 522A-R and the degree of lesion change in left lung 522A-L may be separated. In this case, the degrees of change for the target lesions (e.g., Ptx, PEf, or Csn) may be displayed with graphical indicators, such as icon, and the text describing the degree of change may be omitted or displayed.

The additional SC screen 500A may highlight the lesion changed that needs to be highlighted, for example, the lesion that have `Appeared' or the lesion that have 'Increased', for visual distinction. For example, the additional SC screen 500A may display change information (an icon of `Appeared' or text `Appeared') on the lesion PEf in the SC 512A of the target image because the lesion PEf has appeared in the left lung. Since the newly appeared lesion PEf is a lesion that needs to be highlighted, the newly appeared lesion PEf may be highlighted by giving a visual change (change in color, size, shape, and the like) to the text (PEf or `Appeared') or the change degree icon displayed on the image, or by adding a highlight mark 513A.

Additionally, when there is a change that requires highlighting (e.g., `Appeared' or 'Increased'), the additional SC screen 500A may highlight the change to visually distinguish the change from other degrees of change in the analysis information region 520A. For example, in the degree of lesion change in left lung 522A-L, the change information for lesion PEf (an icon of `Appeared' or text `Appeared') may be colored to stand out.

Referring to FIG. 10, the additional SC screen 500B displayed on the viewer 110 may include an image region 510B and an analysis information region 520B, and may display photographing time points of a previous image and a target image. The arrangement or area size of the additional SC screen 500B may be designed to vary.

The image region 510B may display an SC 511B containing the analysis result of the previous image and an SC 512B containing the analysis result of the target image together. The SCs 511B and 512B of the previous image and the target image may display the results of the analysis (lesion information) of the respective images, and the SC 512B of the target image may further display lesion change information of the target lesions for comparison among the detected lesions.

The analysis information region 520B displays the comparative analysis result (the degree of lesion change) with the previous image. The additional SC screen 500B may display the target lesions selected according to the user's setting in the analysis information region 520B. For example, when the user sets Ptx and Csn for display while setting not to display Ndl and PEf, the analysis information region 520B may only display the degrees of change in Ptx and Csn, the lesions designated for display. In addition, the lesion change information displayed in the SC 512B of the target image may only be provided for the target lesions selected for display.

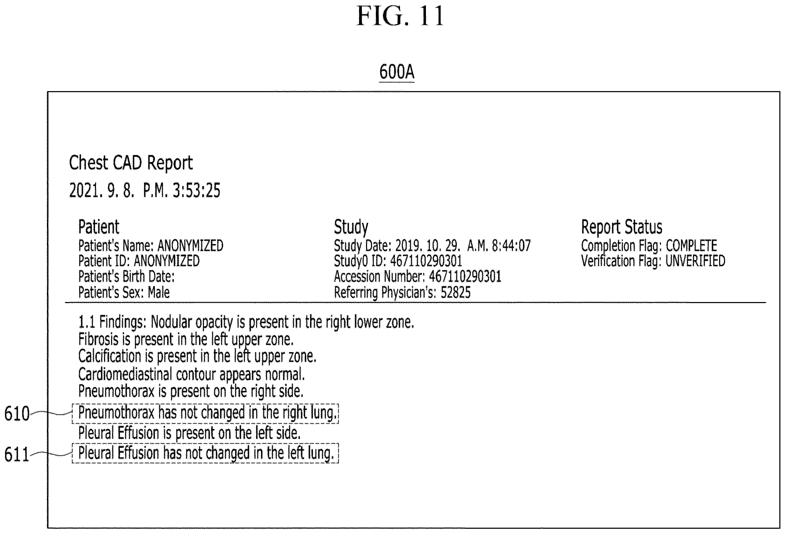

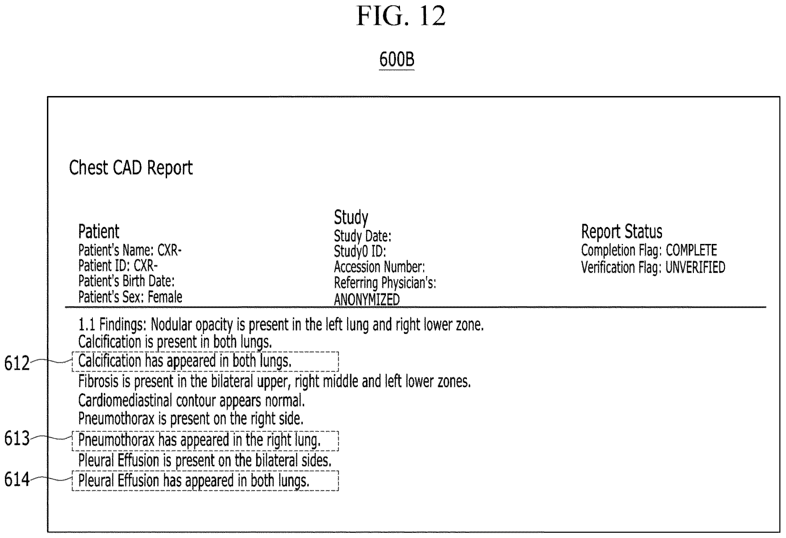

FIGS. 11 and 12 are diagrams illustrating examples of a generated analysis report according to the exemplary embodiment.

Referring to FIGS. 11 and 12, the analysis result of the target image by the image analysis device 300 may be generated as an analysis report. The analysis report may further include the comparative analysis result between the target image and the previous image, with the lesion information detected in the target image.

The analysis report may be, for example, a structured report following the DICOM standard. The analysis report may display the results of the comparative analysis of comparison target lesions A, B, C, and D, such as "A has appeared in the left (or right or both) lung..., B has not changed ..., C has increased..., D has disappeared...".

For example, the analysis report 600A may list the lesions detected in the target image, and may include descriptive statements 610, such as "Pneumothorax has not changed in the right lung.". This description may indicate that a pneumothorax detected in the right lung in the target image has no change compared to the previous image based on the comparative analysis result. Further, when the pleural effusion detected in the left lung of the target image has no change compared to the previous image, the analysis report 600A may include descriptive statements 611, such as "Pleural effusion has not changed in the left lung.".

For example, the analysis report 600B may list the lesions detected in the target image and may include descriptive statements 612, such as "Consolidation has appeared in both lungs.". This description may indicate that a consolidation that were not detected on the previous image is present in the left and right lungs as the comparative analysis result between the target image and the previous image. Further, when a pneumothorax is detected in the right lung of the target image that was not detected in the previous image, the analysis report 600B may include descriptive statements 613, such as "Pneumothorax has appeared in the right lung.". Further, when pleural effusion is detected in the left and right lungs of the target image that was not detected in the previous image, the analysis report 600B may include descriptive statements 614, such as "the pleural effusion has appeared in both lungs.".

Thus, the analysis report may further detail the change in the lesion over time, along with the analysis result including the presence of the lesion. Consequently, users can recognize, whether a lesion is newly detected, remains unchanged, has decreased in size, or has increased in size, solely from the analysis report of the target image without viewing the previous image or the analysis report of the previous image. In particular, the analysis report provides information about lesions that were previously present but disappeared and were not detected. Thus, the analysis report of the target image allows the user to recognize the lesions that disappeared and were not detected in the current image.

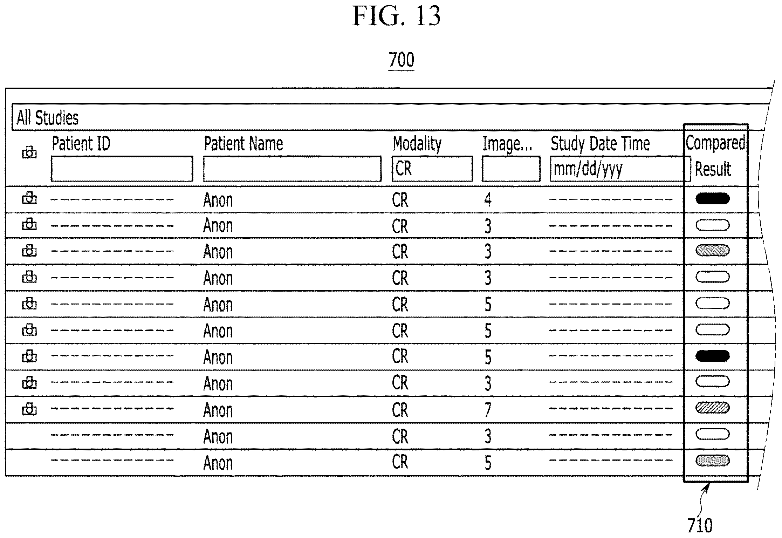

FIG. 13 is a diagram illustrating an example of a generated worklist according to the exemplary embodiment.

Referring to FIG. 13, the viewer 110 executed on the user terminal 100 may provide a worklist 700 in association with the image storage device 200. The worklist 700 lists and presents a list of images assigned for the user's reading task in a tabular format together with key information. The worklist is not necessarily included in the viewer 110 and may be installed as a separate program. The information displayed in the worklist may be provided by the viewer 110 or the image storage device 200, and for ease of description, the information may be described as being provided by the viewer 110.

The viewer 110, in association with the image storage device 200, may display the analysis result analyzed by the image analysis device 300 for the target image in a designated column of the worklist 700. The analysis result may include a result of an AI analysis for the target image and a comparative analysis result between the target image and the previous images. In this case, the viewer 110 may display the comparative analysis results in a designated column 710 of the worklist 700. The viewer 110 may display the comparative analysis results recorded in the DICOM Private tag in the worklist 700. In this case, the viewer 110 may indicate in the worklist 700 whether the current-prior image comparison function is activated based on the Private tag of the DICOM image.

The method of displaying the comparative analysis result in the worklist may be done in a variety of ways, such as color chips, text, icons, or flagging. For example, the viewer 110 may distinguish the comparative analysis result contained in the private tag by a color chip and display the comparative analysis result in a designated column 710. The color chips for the comparative analysis result may be set by the user, or the viewer 110 may display the color chips set based on the priority or importance of the comparative analysis result.

A representative degree of change (e.g., `Appeared' or icon thereof) may be displayed in the designated column 710 of the worklist. Since the comparative analysis result include the degrees of change for multiple lesions, the degree of change with high importance (e.g., `Appeared' or 'Increased') may be determined as the representative degree of change, and the representative degree of change may be displayed in the worklist. Also, since each lesion has different urgency, the degree of change representative for the comparative analysis result between the corresponding image may be determined by considering the importance of the lesion or the importance of the degree of lesion change.

The priority of reading tasks in the worklist may be determined according to the comparative analysis result generated by the image analysis device 300. For example, the user may view the comparative analysis results performed on the images to be read, and sort the order of reading tasks based on the importance of the comparative analysis results through the worklist 700 to increase the efficiency of the reading work. Alternatively, the viewer 110 may provide the worklist by setting a high priority to the image presenting the change (e.g., `Appeared' or 'Increased'), warranting an attention in reading task. For example, when the lesion change included in the comparative analysis result is a positive change such as no change, disappearance of a lesion, or decrease of the lesion, the reading priority of the corresponding image may be set to be low. On the other hand, when the lesion change included in the comparative analysis result is a negative change such as new appearance or increase in the lesion, the reading priority of the corresponding image may be set to be high.

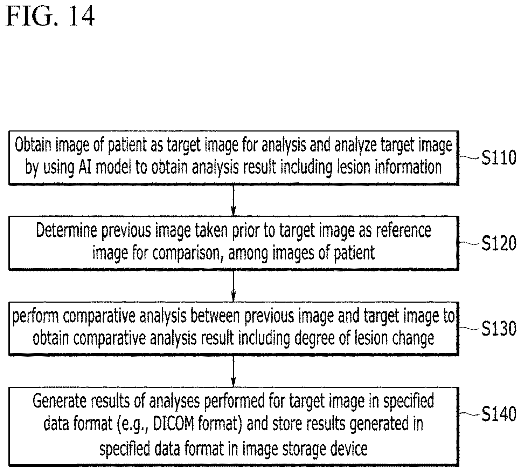

FIG. 14 is a flow diagram of an image analysis method according to the exemplary embodiment.

Referring to FIG. 14, the image analysis device 300 obtains an image of a patient as a target image for analysis and analyzes the target image by using an AI model to obtains an analysis result including lesion information (S1 10). The analysis result may include lesion information including a lesion score, lesion location, and the like, and key medical indicators including an abnormality score.

The image analysis device 300 determines a previous image taken prior to the target image as a reference image for comparison, among the images of the patient (S120). The image analysis device 300 may search for previously taken images in the image storage device 200 based on a Patient ID of the target image, and select a previous image that satisfies a condition. The previous image that is a comparison reference may be determined in a variety of ways. For example, the image analysis device 300 may determine the latest image as the reference image among the previous images of the patient stored in the image storage device 200, determine the previous image taken at a certain time interval from the target image as the reference image, or determine the image on which the AI analysis or reading was performed as the reference image. The time point at which the image analysis device 300 performs the comparative analysis of the target image and the previous image may be varied. For example, the image analysis device 300 may perform the comparative analysis when receiving an analysis request for the target image, or the image analysis device 300 may receive a separate comparative analysis request to perform the comparative analysis.

The image analysis device 300 performs a comparative analysis between the previous image and the target image to obtain a comparative analysis result including the degree of lesion change (S130). The image analysis device 300 may extract lesion information for the at least one comparative target lesion from the analysis result of the previous image and the analysis result of the target image. The image analysis device 300 may determine the degree of change in the target lesion for comparison over time and generate the comparative analysis result including the degree of change in the comparison target lesion. The image analysis device 300 may determine the degree of change by matching the lesions detected at corresponding positions in the previous image and the target image, and may determine that the lesion has disappeared or appeared when the lesion is present only in the previous image or the target image. The image analysis device 300 may determine that a lesion change for a target lesion exists when the target lesion has been changed equal to or greater than a threshold for the target lesion. The threshold may be set differently for different types of lesions. A lesion change may be determined based on, but need not be limited to, a change in the area occupied by the lesion or a change in the abnormality score for the lesion. The degree of lesion change may be analyzed as `No change', 'Disappeared', `Appeared', 'Decreased', or 'Increased', as shown in Table 3. On the other hand, the image analysis device 300 may analyze the previous image by using an AI model for the comparative analysis of the previous image and the target image, obtain an analysis result including lesion information of the previous image, and compare the obtained analysis result with the analysis result of the target image. Alternatively, when there is an AI analysis result for the identified previous image, that is, when the image analysis device 300 has already undergone the previous image analysis, the image analysis device 300 may directly compare the analysis result of the previous image and the analysis result of the target image without having to perform the analysis of the previous image again.

The image analysis device 300 generates the results of analyses performed for the target image in a specified data format (e.g., DICOM format) and stores the results generated in the specified data format in the image storage device 200 (S140). For example, the image storage device 200 may store the analysis result including lesion information of the target image as a secondary capture of the DICOM standard, which may be referred to as a default SC. The image storage device 200 may archive the comparative analysis result between the target image and the previous image as a secondary capture in the DICOM standard, which may be stored as an augmented SC with the results of the comparative analysis appended to the analysis result of the target image, or as an additional SC showing a comparison of the analysis result of the previous image and the analysis result of the target image. The analysis results performed on the target image may be embedded in the private tag of the DICOM image. The analysis results performed on the target image may be displayed sequentially in the viewer 110 while being grouped as the secondary capture of the target image. The analysis results performed for the target image, in particular, the results of the comparative analyses, may be displayed in a worklist. Furthermore, the analysis results performed on the target image, in particular the degrees of lesion change, may be displayed in an analysis report in the form of sentences.

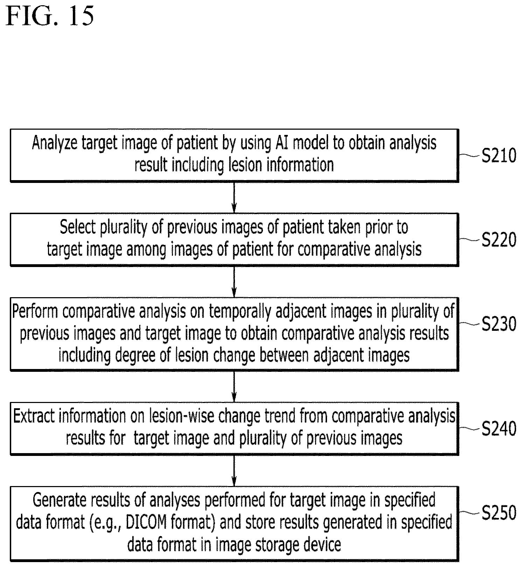

FIG. 15 is a flow diagram of an image analysis method according to another exemplary embodiment.

Referring to FIG. 15, the image analysis device 300 analyzes a target image of a patient by using an AI model to obtain an analysis result including lesion information (S210). The analysis result may include lesion information including a lesion score, lesion location, and the like, and key medical indicators including an abnormality score. The image analysis device 300 may automatically generate analysis results when obtaining a new image and provide analysis results for the new image of the patient when a patient ID is selected in a PACS worklist or viewer.

The image analysis device 300 selects a plurality of previous images of the patient taken prior to the target image among the images of the patient for the comparative analysis (S220). The number N of medical images to be used in the comparative analysis and an image selecting method may be pre-set by user's configuration or may be adaptively determined during the progress course of the comparative analysis.

The image analysis device 300 performs a comparative analysis on temporally adjacent images in the plurality of previous images and the target image to obtain comparative analysis results including the degree of lesion change between the adjacent images (S230). The degree of lesion change may be analyzed as `No change', 'Disappeared', `Appeared', 'Decreased', or 'Increased', as shown in Table 3.

The image analysis device 300 extracts information on a lesion-wise change trend from the comparative analysis results for the target image and the plurality of previous images (S240). The information indicating the change trend by lesion may be expressed as a change rate, a change graph over time according to date of acquisition, a table, or the like.

The image analysis device 300 generates the results of the analyses performed for the target image in a specified data format (e.g., DICOM format) and stores the results generated in the specified data format in the image storage device 200 (S250). For example, the analysis results including the lesion information of the target image may be stored as a secondary capture in the DICOM standard, and a plurality of the comparative analysis results may be stored as a secondary capture in the DICOM standard. Each comparative analysis result may be stored as an additional SC showing the two compared images together, or an augmented SC. The results of the analysis performed on the target image may be embedded in the private tag of the DICOM image. The analysis results performed on the target image may be displayed sequentially in the viewer 110 while being grouped as the secondary capture of the DICOM image. The analysis results performed for the target image, in particular the results of the comparative analyses, may be displayed in a worklist. Further, the analysis results performed on the target image, in particular, the degree of lesion change, may be displayed in an analysis report in the form of sentences.

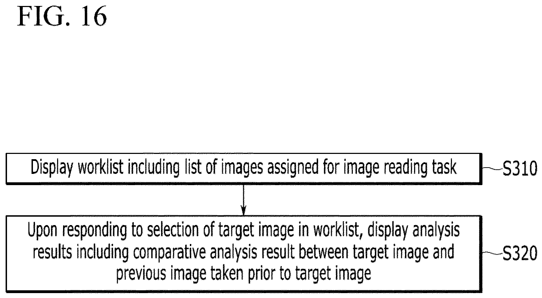

FIG. 16 is a flow diagram of a method of providing an analysis result according to an exemplary embodiment.

Referring to FIG. 16, the viewer 110, in association with the image storage device 200, displays a worklist including a list of images assigned for image reading task (S310). The viewer 110 displays the analysis result of each image analyzed by the image analysis device 300 in a designated column of the worklist, which may include the result of the AI analysis of the image and the comparative analysis result with the previous image. The viewer 110 may display the comparative analysis result in the worklist in a variety of ways (e.g., color chips, text, flagging, or icons). For example, the viewer 110 may distinguish the comparative analysis results contained in the private tag of the image by color chips and display the comparative analysis results in the worklist. The viewer 110 may display the degree of change (e.g., `Appeared' or 'Increased') representing the comparative analysis result in the worklist. The viewer 110 may display a list of images in the worklist sorted according to a priority determined by the comparative analysis result.

The viewer 110, upon responding to the selection of a target image in the worklist, displays the analysis results including the comparative analysis result between the target image and previous image taken prior to the target image (S320). The viewer 110 may retrieve and display the analysis results stored in a specified data format (e.g., DICOM format). When the viewer 110 is set to provide the comparative analysis result using an augmented SC, the viewer 110 may sequentially display the taken image of the target image and the augmented SC including the analysis result of the target image with the comparative analysis result. When the viewer 10 is set to provide the comparative analysis result using an additional SC, the viewer 110 may sequentially display the taken image of the target image, a default SC including the analysis result of the target image, and an additional SC including the comparison result between the target image and the previous image.

The viewer 110 is implemented as a computer program stored on a computer-readable recording medium and includes instructions executable by a processor. The computer program may include instructions that cause the processor executing the computer program, in association with the image storage device 200, to display a worklist including a list of images assigned for an image reading task and, when a target image is selected in the worklist, to display analysis results including the comparative analysis results between the target image and the previous image. The computer program may include instructions to display the results of the comparative analysis of the target image and the previous image in the worklist. The computer program may include instructions to display an augmented SC in which the comparative analysis result is appended to the default SC having the analysis result of the target image, or to display an additional secondary capture which is provided independently with the default SC.

Here, the user does not necessarily have to go through a worklist to check the results of the analysis including the results of the comparative analysis of the target image and the previous image, but this is an illustrative exemplary embodiment. For example, a user (e.g., a doctor) may check the analysis results including the comparative analysis results between the target image and the previous image, through the viewer that displays the patient's medical information.

The terminal or devices 100, 200, and 300 configuring the medical imaging system 1 of the present disclosure may include one or more processors, a memory for loading a computer program executed by the processor, a storage device for storing the computer program and various data, and a communication interface. In addition, the terminal or devices 100, 200, and 300 may further include various other components. A processor is a device that controls the operation of a server and may be various forms of processor that processes instructions contained in a computer program, and may include, for example, at least one of a Central Processing Unit (CPU), a Micro Processor Unit (MPU), a Micro Controller Unit (MCU), a Graphic Processing Unit (GPU), or any other form of processor well known in the art of the present disclosure. The memory stores various data, commands, and/or information. The memory may be implemented to store instructions such that the instructions described to execute the operations of the present disclosure are processed by the processor. The memory may be, for example, Read Only Memory (ROM) and Random Access memory (RAM). The storage device may store computer programs and other data on a non-transitory basis. The storage device may include a non-volatile memory, such as a Read Only Memory (ROM), an Erasable Programmable ROM (EPROM), an Electrically Erasable Programmable ROM (EEPROM), a flash memory, or the like, a hard disk, a removable disk, or any other form of computer-readable recording medium well known in the art to which the present disclosure belongs. The communication interface may be a wired/wireless communication module supporting wired/wireless communication. The computer program includes instructions executable by a processor and is stored on a non-transitory computer readable storage medium, and the instructions cause the processor to perform the operations of the present disclosure.

The exemplary embodiments of the present disclosure described above are not only implemented through the apparatus and method, but may also be implemented through programs that realize functions corresponding to the configurations of the exemplary embodiment of the present disclosure, or through recording media on which the programs are recorded.

Although an exemplary embodiment of the present disclosure has been described in detail, the scope of the present disclosure is not limited by the exemplary embodiment. Various changes and modifications using the basic concept of the present disclosure defined in the accompanying claims by those skilled in the art shall be construed to belong to the scope of the present disclosure.

## Claims

1. A device for analyzing an image, the device comprising:
a memory; and
a processor for executing instructions stored in the memory,
wherein the processor is configured to:
analyze a target image of a patient by using an artificial intelligence model to obtain an analysis result including lesion information,
determine a previous image taken prior to the target image as a reference image, among images of the patient, and
perform a comparative analysis on the reference image and the target image to obtain a comparative analysis result including a degree of lesion change.

2. The device of claim 1, wherein the processor is configured to determine an image satisfying a condition among previous images of the patient as the reference image.

3. The device of claim 1, wherein the processor is configured to:
extract lesion information on at least one target lesion for comparison from an analysis result of the reference image and the analysis result of the target image,
determine the degree of change for each target lesion over time based on the extracted lesion information, and
generate the comparative analysis result including the degree of change for each target lesion.

4. The device of claim 3, wherein the processor is configured to:
determine the degree of change for each target lesion as one of No change, Disappeared, Appeared, Decreased, Increased, and No existence.

5. The device of claim 3, wherein the processor is configured to
determine that a lesion change for a target lesion exists when the target lesion has been changed equal to or greater than a threshold for the target lesion.

6. The device of claim 5, wherein the processor is configured to determine the lesion change by using a threshold set for each target lesion.

7. The device of claim 1, wherein the processor is configured to:
select a plurality of previous images taken prior to the target image as a plurality of reference images, and
perform a comparative analysis on temporally adjacent images in the plurality of reference images and the target image to obtain a plurality of comparative analysis results including the degree of lesion change between the adjacent images.

8. The device of claim 1, wherein the processor is configured to
generate results of the analyses performed for the target image in a specified data format and store the results generated in the specified data format in an image storage device.

9. The device of claim 8, wherein the processor is configured to
generate the comparative analysis result as a secondary capture of a DICOM standard.

10. The device of claim 9, wherein the processor is configured to
generate an augmented secondary capture in which the comparative analysis result is appended to the analysis result of the target image.

11. The device of claim 9, wherein the processor is configured to
generate an additional secondary capture that shows a comparison between an analysis result of the reference image and the analysis result of the target image.

12. A method of operating an image analysis device, the method comprising:
analyzing a target image of a patient by using an artificial intelligence model to obtain an analysis result including lesion information; and
determining a previous image taken prior to the target image as a reference image, among images of the patient; and
performing a comparative analysis on the reference image and the target image to obtain a comparative analysis result including a degree of lesion change.

13. The method of claim 12, wherein the performing the comparative analysis includes:
extracting lesion information on at least one target lesion for comparison from an analysis result of the reference image and the analysis result of the target image;
determining the degree of change for each target lesion over time based on the extracted lesion information; and
generating the comparative analysis result including the degree of change for each target lesion.

14. The method of claim 13, wherein the determining the degree of change includes determining the degree of change for each target lesion as one of No change, Disappeared, Appeared, Decreased, Increased, and No existence.

15. The method of claim 12, further comprising:
generating results of the analyses performed for the target image in a specified data format including a secondary capture of the DICOM standard,
wherein the generating the results of the analyses includes:
generating an augmented secondary capture in which the comparative analysis result is appended to the analysis result of the target image, or
generating an additional secondary capture that shows a comparison between an analysis result of the reference image and the analysis result of the target image.
